# EUROPÄISCHE PATENTSCHRIFT

(11) **EP 0 587 072 B1**
(45) Veröffentlichungstag und Bekanntmachung des Hinweises auf die Patenterteilung: **07.01.1998**
(21) Anmeldenummer: 93114184.0
(22) Anmeldetag: 04.09.1993
(51) Int. Cl.: C07C 51/58, C07C 59/58, C07D 309/28

(54) **Verfahren zur Herstellung von alpha,beta-ungesättigten beta-Oxycarbonsäurechloriden**
Process for the preparation of alpha, beta-unsaturated beta-oxycarboxylic acid chlorides
Procédé de préparation de chlorures d'acides bêta oxycarboxyliques insaturés en position alpha, bêta

(30) Priorität: 10.09.1992 DE 4230283
(43) Veröffentlichungstag der Anmeldung: 16.03.1994
(73) Patentinhaber: BASF Aktiengesellschaft, 67063 Ludwigshafen (DE)
(72) Erfinder: Henkelmann, Jochem, Dr., D-6800 Mannheim 1 (DE); Tietze, Lutz, Prof. Dr., D-3400 Goettingen (DE)

(56) Entgegenhaltungen:
- DE-B- 1 073 479
- US-A- 2 768 174
- US-A- 4 966 995

## Beschreibung

Die vorliegende Erfindung betrifft ein Verfahren zur Herstellung von alpha,beta-ungesättigten beta-Oxycarbonsäurechloriden der Formel I 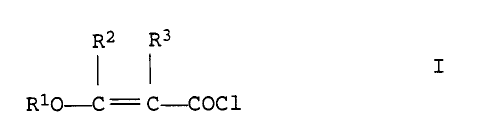 in der R¹ für eine gegebenenfalls substituierte Alkylgruppe, Cycloalkylgruppe, Alkenylgruppe, Cycloalkenylgruppe, Arylgruppe, Heterocyclylgruppe oder Heteroarylgruppe und R² und R³ unabhängig voneinander für Wasserstoff oder eine gegebenenfalls substituierte Alkylgruppe, Cycloalkylgruppe, Alkenylgruppe, Cycloalkenylgruppe, Alkinylgruppe, Arylgruppe, Heterocyclylgruppe oder Heteroarylgruppe stehen, dadurch gekennzeichnet, daß man an ein Enolderivat der Formel II 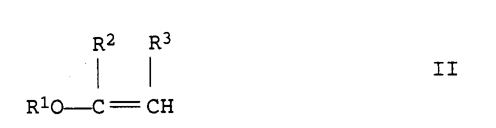 in Abwesenheit eines tertiären Amins bei 20 bis 60°C eine Verbindung IIIa, IIIb oder IIIc 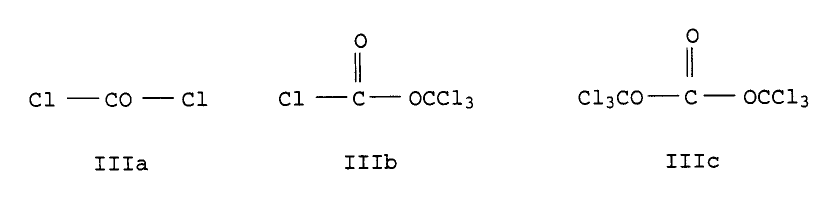 addiert und das so erhaltene Säurechlorid der Formel IV 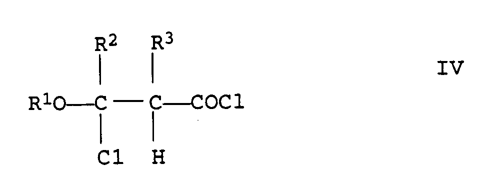 durch Eliminierung von Chlorwasserstoff (HCI) bei 30 bis 80°C zu I umsetzt.

Alpha,beta-ungesättigte beta-Oxycarbonsäuren und deren Derivate sind wertvolle Zwischenprodukte in der organischen Synthese (Heterocycles 16, 1515 (1981); Can. J. Chem. 63, 2787 (1985); J. Med. Chem. 26, 1075 (1983); J. Heter. Chem. 13, 1015 (1976); J. Chem. Soc., Perkin Trans. I. 1241 (1976); Aust. J. Chem. 30, 459 (1977); Zhur. org. Khim. 2, 66 (1966); Can. J. Chem. 44, 661 (1966)).

Man erhält sie beispielsweise durch Esterkondensation (zum beta-Ketoderivat) und anschließende Enolisierung gemäß dem folgenden Reaktionsschema: 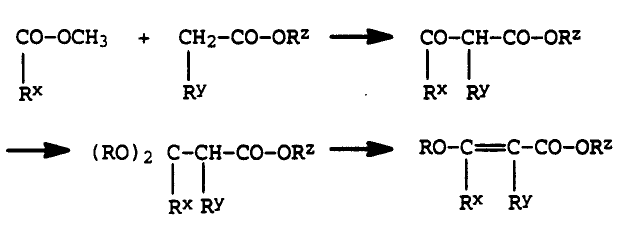 (z.B. Zhur. org. Khim., 2, 66 (1966)

Eine weitere Möglichkeit ist die Umsetzung von Orthoformiat mit alpha-Halogencarbonsäureestern und anschließende Eliminierung: 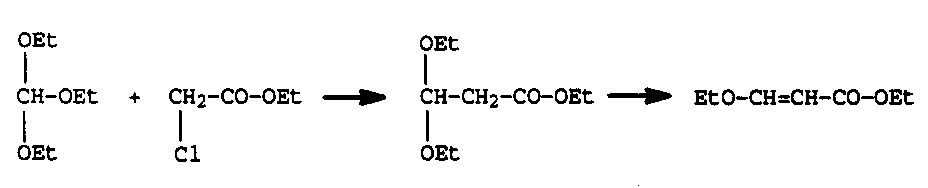 (z.B. J. Med. Chem., 26, 1075-1076 (1983)).

Eine weitere Möglichkeit ist die Umsetzung von Orthoformiat mit Keten und anschließende Eliminierung: 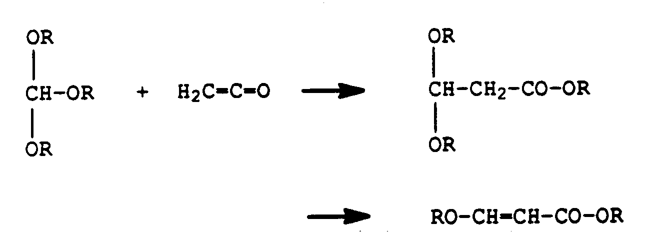 (R= z.B. Me, Et)
(DK-A 158462 (1990) = CA 113, 190761W (1990); F. Sorm und J. Smrt, Chem. Listy 47, 413-17 (1953) = CA 49, 175c (1955); D.G. Crosby und R.V. Berthold, J. Org. Chem., 27, 3083-85 (1962); US-A 2,449,471 (1948), = CA 43, 1055 (1949))

Eine weitere Möglichkeit besteht in der Kondensation von Essigsäureestern mit CO und anschließender Alkylierung der Kondensationsprodukte: 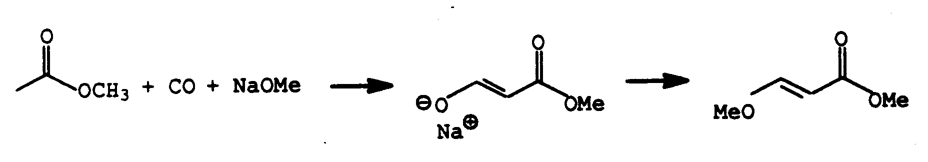 (DE-A 34 15 475)

Aktivierte Derivate der α, β-ungesättigten β-Oxicarbonsäuren, z.B. die Chloride, können durch Hydrolyse der Ester und Chlorierung des Natriumsalzes der Säure 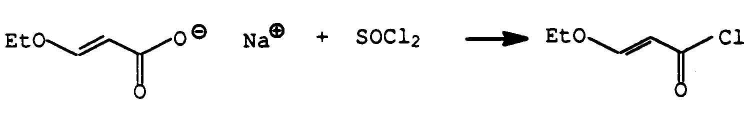 (J. Chem. Soc., 1959, 1169-78) 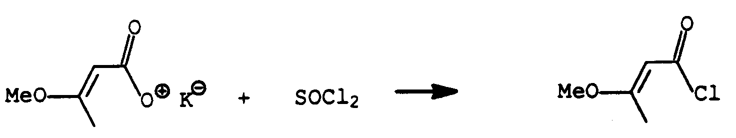 (J. Org. Chem., 1962, 27, 3317-3319)
bzw. durch Chlorierung der freien Säure 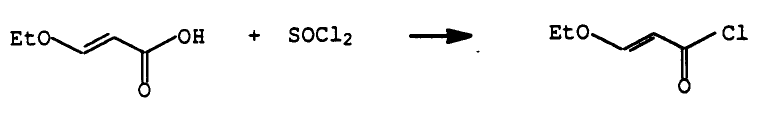 (US-A 4,239,888)
hergestellt werden.

Ein wesentlicher Nachteil der bekannten Methoden beruht darauf, daß nur durch aufwendige (mehrstufige) Synthesen die erhaltenen Ester in aktivierte Carbonsäurederivate wie Halogenide überführt werden können.

Eine direkte Herstellmöglichkeit besteht in der Umsetzung von Vinylethern mit Phosgen: 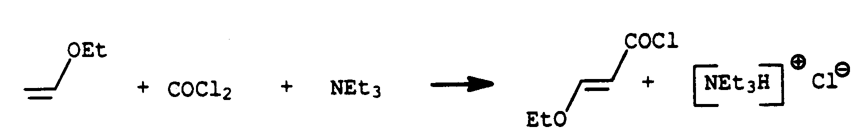 (US-A 2,768,174, SU-A 462,145)

Anstelle von Triethylamin können auch andere Stickstoffbasen, wie z.B. Pyridin oder Diethylanilin, eingesetzt werden. Der während der Reaktion gebildete Chlorwasserstoff kann auch durch Addition an Allylchlorid gebunden werden (SU-A 462 145). Die Reaktion verläuft nicht selektiv. Als Hauptnebenprodukt entsteht das Additionsprodukt von HCl an die jeweiligen Vinylether (US-A 2,768,174).

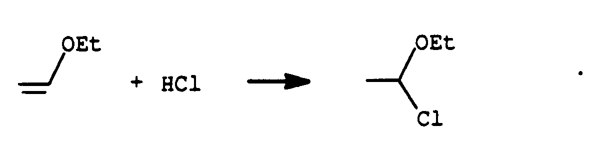

Das gebildete Aminhydrochlorid muß als Feststoff aufwendig abgetrennt werden, das bei der Abfangreaktion von HCl mit Allylchlorid gebildete Dichlorpropan ist aufwendig zu entsorgen.

Des weiteren neigen Gemische aus Vinylethern und Phosgen zur spontanen Polymerisation (US-A 2,464,747).

Außerdem gestaltet sich die Herstellung von Carbonsäuren, anderen Estern und Amiden infolge der benötigten Umsetzungsbedingungen bei diesen Reaktionen insbesondere im Hinblick auf thermisch instabile Reste schwierig, weshalb die bekannten Verfahren einen breiten Zugang zu derartigen Verbindungen nicht zulassen.

Aus der Literatur ist weiterhin bekannt, daß Oxalylchlorid bei Raumtemperatur an zwei Äquivalente Vinylether addiert: 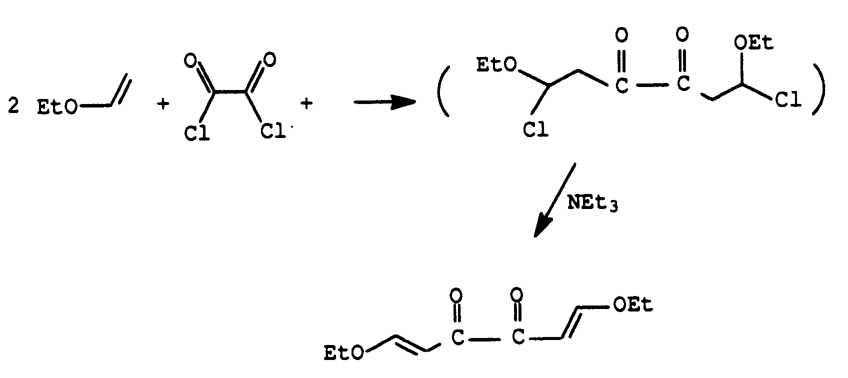 (Chem. Ber. 98, 2260-2265 (1965))

Das zunächst gebildete Bisadditionsprodukt ist thermisch instabil und zersetzt sich spontan. Es kann durch Eliminierung mit Triethylamin in das stabile 1,4-Bis-ethoxymethylen-butandion-(2,3) überführt werden.

In gleicher Weise reagieren auch andere Vinylether mit Oxalylchlorid, z.B. 5,6-Dihydro-4H-pyran.

Aus der US 2,436,645 ist ein Verfahren zur Herstellung von 2,3 Dihydropyrancarbonsäurechlorid bekannt. Nach dieser Lehre setzt man Phosgen und Dihydropyran bei einer Temperatur, die zwischen 15°C und dem Gefrierpunkt des Dihydropyrans liegt, zum Chlortetrahydropyrancarbonsäurechlorid um.

Durch Erwärmung oberhalb der Atmosphärentemperatur wird aus diesem Zwischenprodukt durch Elimination von HCL 2,3-Dihydropyrancarbonsäurechlorid gebildet. Die Ausbeuten können jedoch noch nicht vollständig befriedigen.

Aufgabe der vorliegenden Erfindung war es, alpha,beta-ungesättigte beta-Oxicarbonsäuren bzw. deren aktivierten Derivate, insbesondere deren Halogenide, auf technisch einfache und wirtschaftliche Weise zugänglich zu machen.

Entsprechend dieser Aufgabe wurde ein Verfahren zur Herstellung von alpha,beta-ungesättigten beta-Oxycarbonsäurechloriden der Formel I, 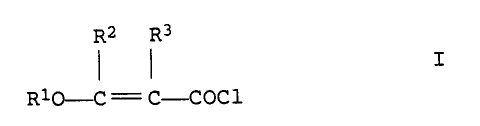 in der R¹ für eine gegebenenfalls substituierte Alkylgruppe, Cycloalkylgruppe, Alkenylgruppe, Cycloalkenylgruppe, Arylgruppe, Heterocyclylgruppe oder Heteroarylgruppe und R² und R³ unabhängig voneinander für Wasserstoff oder eine gegebenenfalls substituierte Alkylgruppe, Cycloalkylgruppe, Alkenylgruppe, Cycloalkenylgruppe, Alkinylgruppe, Arylgruppe, Heterocyclylgruppe oder Heteroarylgruppe
stehen, gefunden, welches dadurch gekennzeichnet ist, daß man an ein Enolderivat der Formel II 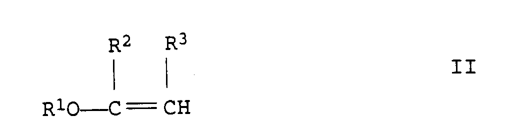 in Abwesenheit eines tertiären Amins bei 20 bis 60°C eine Verbindung IIIa, IIIb oder IIIc 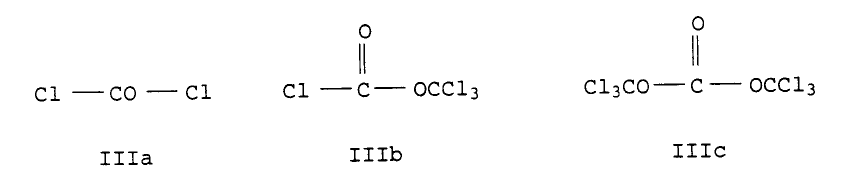 addiert und das so erhaltene Säurechlorid der Formel IV 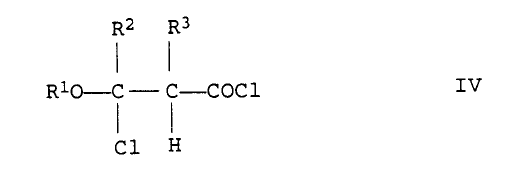 durch Eliminierung von Chlorwasserstoff (HCl) bei 30 bis 80°C zu I umsetzt.

Die Umsetzung kann ohne Verwendung von Lösungs- bzw. Verdünnungsmitteln durchgeführt werden, sofern die Umsetzungspartner bei der Umsetzungstemperatur flüssig sind. Es ist aber auch möglich, die Umsetzung in einem aprotischen Lösungs- oder Verdünnungsmittel durchzuführen.

Geeignete Lösungs- oder Verdünnungsmittel sind beispielsweise aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, sowie Ether wie Diethylether, Diisopropylether, tert.-Butylmethylether, Dioxan, Anisol und Tetrahydrofuran, und Nitrile wie Acetonitril und Propionitril.

Es können auch Gemische der genannten Lösungsmittel verwendet werden.

Besonders bevorzugt führt man die Umsetzung ohne Lösungsmittel oder in aromatischen Kohlenwasserstoffen wie Toluol als Lösungsmittel durch.

Die Umsetzungspartner II und III werden im allgemeinen im Verhältnis von 0,1:1 bis 1:1 mol II/IIIa, IIIb bzw. IIIc, vorzugsweise 0,2:1 bis 0,8:1 mol II/IIIa, IIIb bzw. IIIc, insbesondere 0,4:1 bis 0,6:1 mol II/IIIa, IIIb bzw. IIIc, miteinander umgesetzt.

Da sowohl die Halogenide III als auch das gebildete Säurechlorid IV gegen Feuchtigkeit labil sind, empfiehlt es sich, die Umsetzung unter Ausschluß von Wasser, vorzugsweise unter Schutzgasatmosphäre (Stickstoff oder ein anderes Inertgas) durchzuführen.

Im Fall der Umsetzung von II mit IIIb bzw. IIIc kann es von Vorteil sein, die Reaktion durch die Zugabe von katalytischen Mengen eines tertiären Amins wie Triethylamin und Pyridin zu beschleunigen.

Das so gewonnene Säurechlorid IV spaltet bei 30 bis 80°C Chlorwasserstoff (HCl) ab, wobei sich das entsprechende alpha,beta-ungesättigte beta-Oxycarbonsäurechlorid I bildet.

Für diese Stufe der Umsetzung kann es von Vorteil sein, den gebildeten Chlorwasserstoff aus dem Reaktionsvolumen zu entfernen, sei es durch leichten Unterdruck oder dadurch, daß man Inertgas durch die Reaktionsmischung oder das Reaktionsgefäß leitet und somit den gebildeten Chlorwasserstoff entfernt.

Das überschüssige Chlorid der Formel IIIa, IIIb bzw. IIIc kann in die Synthese rückgeführt werden (Phosgen) und muß in jedem Fall zur Isolierung des reinen Wertproduktes abgetrennt werden. Gleiches gilt für evtl. zugesetzte Katalysatoren.

Die so erhaltenen Reaktionsgemische werden in üblicher Weise durch Destillation aufgearbeitet.

Sie können aber auch direkt (ohne weitere Reinigung) in an sich bekannter Weise zu Carbonsäuren, Estern oder Amiden umgesetzt werden:

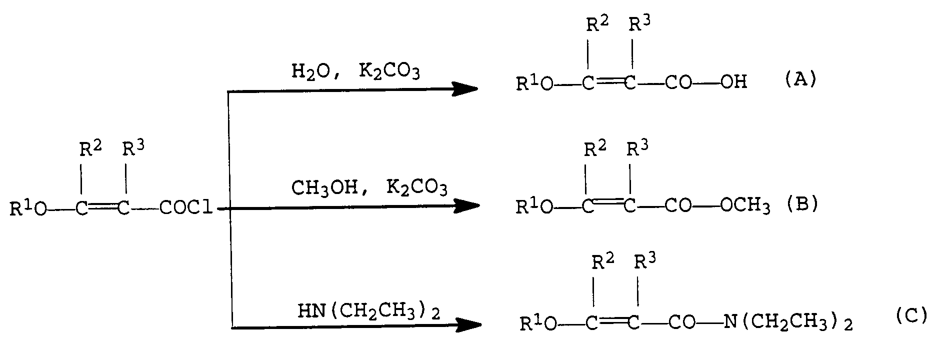

Die Umsetzungen (A), (B) und (C) sind allgemein bekannt (z.B. Houben-Weyl, 4. Auflage, Bd. 8/3, S. 425-427; Houben-Weyl, Bd. 8/3, S. 543-547; Houben-Weyl, Bd. 8/3, S. 655-658 (Stuttgart 1952); US-A 4,239, 888).

Augrund einer trans-Eliminierung von Chlorwasserstoff fallen die nach dem neuen Verfahren erhältlichen alpha,beta-ungesättigten beta-Oxycarbonsäurechloride vorwiegend als E-Isomere der Doppelbindung (R¹O-Gruppe zu Carbonylgruppe) an.

Die Carbonsäurechloride IIIa, IIIb und IIIc sind bekannt. Im allgemeinen wird aus wirtschaftlichen Erwägungen der Verbindung IIIa (Phosgen) der Vorzug gegeben.

Als Enolkomponente der Formel II finden Verbindungen Verwendung, in denen die Reste die folgende Bedeutung haben:
R¹
   eine ggf. subst. Alkylgruppe, Cycloalkylgruppe, Alkenylgruppe, Cycloalkenylgruppe, Arylgruppe, Heterocyclylgruppe oder Heteroarylgruppe;
R² und R³ unabhängig voneinander
   Wasserstoff oder eine ggf. subst. Alkylgruppe, Cycloalkylgruppe, Alkenylgruppe, Cycloalkenylgruppe, Alkinylgruppe, Arylgruppe, Heterocyclylgruppe oder Heteroarylgruppe.

Nach den bisherigen Erkenntnissen haben Substituenten an den vorstehend genannten C-organischen Resten keinen Einfluß auf den Verlauf der Umsetzung. Im allgemeinen kommen als Substituenten der C-organischen Reste solche in Betracht, die selbst keine basischen oder sauren Eigenschaften haben, beispielsweise Halogenatome, Nitrogruppen, Cyanogruppen, Estergruppen, Carbonylgruppen, Ether- oder Thioethergruppen.

Insbesondere sind unter den C-organischen Resten die folgenden zu verstehen:
C₁-C₈-Alkyl, besonders C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
C₃-C₈-Alkenyl, besonders C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
C₃-C₈-Alkinyl, besonders C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl,1-Ethyl-3-butinyl, 2-Ethyl-3-butinyl und 1-Ethyl-1-methyl-2-propinyl;
C₃-C₈-Cycloalkyl wie Cyclopropyl, Cyclobutyl, Cyclopentyl, Cyclohexyl, Cycloheptyl und Cyclooctyl;
C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl;
ein 3- bis 8-gliedriges, gesättigtes oder einfach ungesättigtes Ringsystem, welches neben Kohlenstoff-Ringgliedern ein bis drei Heteroatome aus der Gruppe Sauerstoff und Schwefel enthalten kann, vorzugsweise 5- bis 6-gliedrige, gesättigte oder ungesättigte Heterocyclen, enthaltend ein bis drei Sauerstoff- oder Schwefelatom wie 2-Tetrahydrofuranyl, 3-Tetrahydrofuranyl, 2-Tetrahydrothienyl, 3-Tetrahydrothienyl, Tetrahydrotriazin-3-yl,
Aryl wie Phenyl, 1-Naphthyl oder 2-Naphthyl;
Heteroaryl wie 5-gliedrige aromatische Ringe, welche neben Kohlenstoff-Ringgliedern ein Sauerstoff- oder ein Schwefelatom enthalten, wie 2-Furyl, 3-Furyl, 2-Thienyl und 3-Thienyl.

Die vorstehend genannten C-organischen Reste können ihrerseits partiell oder vollständig halogeniert sein, d.h. an Kohlenstoff gebundene Wasserstoffatome können durch Halogenatome wie Fluor, Chlor, Brom und Jod, vorzugsweise Fluor, Chlor und Brom, insbesondere Fluor und Chlor, ersetzt sein. Diese C-organischen Reste können zusätzlich oder statt dessen auch ein bis drei der folgenden Substituenten tragen:
Cyano; Nitro;
C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio;
C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;
C₃-C₈-Cycloalkyl wie Cyclopropyl, Cyclobutyl, Cyclopentyl, Cyclohexyl, Cycloheptyl und Cyclooctyl;
C₃-C₈-Cycloalkoxy wie Cyclopropyloxy, Cyclobutyloxy, Cyclopentyloxy, Cyclohexyloxy, Cycloheptyloxy und Cyclooctyloxy; C₃-C₈-Cycloalkylthio wie Cyclopropylthio, Cyclobutylthio, Cyclopentylthio, Cyclohexylthio, Cycloheptylthio und Cyclooctylthio:
Phenyl, welches partiell oder vollständig halogeniert sein kann und welchen daneben ein bis drei der folgenden Reste tragen kann: Cyano, Nitro, C₁-C₄-Alkyl wie vorstehend genannt;
C₁-C₄-Halogenalkyl wie vorstehend genannt;
C₁-C₄-Alkoxy wie vorstehend genannt;
C₁-C₄-Halogenalkoxy wie vorstehend genannt;
C₁-C₄-Alkylthio wie vorstehend genannt;
C₁-C₄-Halogenalkylthio wie vorstehend genannt.

Die cyclischen C-organischen Reste können des weiteren ein bis drei der folgenden Substituenten tragen:
C₁-C₄-Alkyl wie Methyl, Ethyl, Propyl, 1-Methylethyl, Butyl, 1-Methylpropyl, 2-Methylpropyl und 1,1-Dimethylethyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl.

Die Verbindungen I sind wertvolle Zwischenprodukte für die Synthese von Wirkstoffen auf dem Gebiet der Pharmaka und des Pflanzenschutzes, für die Synthese von Farbstoffen oder für die Synthese von Polymeren (z.B. J. Org. Chem., 27, 3083 - 3085 (1965); J. Heterocycl. Chem., 13, 1015 - 1020 (1976); US-A 4,239,888; J. Org. Chem., 27, 3317 - 3319 (1965); J. Chem. Soc., 1959, 1169 - 1178).

### Beispiele

### Beispiel 1

### 3-Ethoxyacrylsäurechlorid

Zu einer Lösung aus 72 g (1 mol) Ethylvinylether in 100 g Toluol wurden 110 g (1,1 mol) Phosgen bei 35°C innerhalb von 1,5 h eingeleitet. Anschließend wurde 4 Stunden bei 60°C nachgerührt. Während der gesamten Versuchszeit wurden Phosgen und Ethylvinylether mit einem -78°C-Trockeneiskühler in die Reaktionsmischung rückkondensiert. Anschließend wurde die Lösung bei Raumtemperatur phosgenfrei gestrippt und das Lösungsmittel destillativ entfernt. Nach Vakuumdestillation bei 36°C/0,4 mbar wurden 88 g (66 %) Wertprodukt erhalten.

### Beispiel 2

### 3,4-Dihydropyran-3-carbonsäurechlorid

In eine Lösung von 84 g (1 Mol) 5,6-Dihydro-4H-pyran in 50 g Toluol wurden bei 45-50°C 125 g (1,25 Mol) Phosgen innerhalb von 2,5 Stunden eingegast. Anschließend wurde 5 Stunden bei 60°C nachgerührt. Die Aufarbeitung erfolgte analog Beispiel 1. Nach Vakuumdestillation bei 76°C/1 mbar wurden 103,9 g (80 %) Wertprodukt erhalten.

### Beispiel 3

Der unter Beispiel 2 beschriebene Versuch wurde wiederholt. Das Phosgen wurde bei 60°C innerhalb von 6 Stunden eingeleitet. Anschließend wurde 2 Stunden bei 60°C nachgerührt. Man erhielt nach Aufarbeitung 118,2 g (91 %) Wertprodukt.

### Beispiel 4

### 3-Methoxy-3-methylacrylsäurechlorid

In eine Lösung von 36 g (0,5 Mol) 2-Methoxypopen in 90 g Toluol wurden innerhalb von 1,5 Stunden 70 g (0,7 mol) Phosgen bei 60°C eingeleitet. Anschließend wurde 2 Stunden bei 60°C nachgerührt. Nach destillativer Aufarbeitung bei 50°C/3 mbar wurden 55,8 g (83 %) Wertprodukt erhalten.

### Beispiel 5

### 3-Cyclohexyloxyacrylsäurechlorid

In eine mit -78°C ausgestattete Rührapparatur wurden 50 g (0,5 mol) Phosgen einkondensiert. Anschließend wurde innerhalb von 3 Stunden 50,5 g (0,4 mol) Cyclohexylvinylether bei 20°C eingetropft. Anschließend wurde 5 Stunden bei 50°C nachgerührt. Das überschüssige Phosgen wurde mit Stickstoff ausgetrieben und das Rohprodukt destillativ aufgearbeitet. Bei 110°C/2,5 mbar wurden 66,4 g (88 %) Wertprodukt erhalten.

### Beispiel 6

### 3-Cyclohexyloxyacrylsäurechlorid

Zu 13,5 g (0,068 mol) Chlorameisensäuretrichlormethylester ("Diphosgen") wurden bei Raumtemperatur 12,5 g (0,1 mol) Cyclohexylvinylether innerhalb von 45 Minuten zugetropft. Es wurde 2,5 Stunden bei Raumtemperatur nachgerührt und anschließend 5 Stunden auf 50°C erhitzt. Das bei der Reaktion gebildete uberschüssige Phosgen wurde mit Stickstoff ausgetrieben und das Rohprodukt destillativ aufgearbeitet. Bei 110°C/2,5 mbar wurden 13,2 g (70 %) Wertprodukt erhalten.

### Beispiel 7

### 3-Cyclohexyloxyacrylsäurechlorid

Analog Beispiel 6 wurden 30 g (0,1 mol) Bis-(trichlormethyl)-carbonat ("Triphosgen") in 50 ml Toluol gelöst, mit 34 g (0,27 mol) Cyclohexylvinylether bei 15°C innerhalb von 45 Minuten umgesetzt. Danach wurde 2 Stunden bei Raumtemperatur nachgerührt und anschließend 5 Stunden auf 50°C erhitzt. Das bei der Reaktion gebildete überschüssige Phosgen wurde mit Stickstoff ausgetrieben und das Lösungsmittel destillativ entfernt. Danach wurde das Wertprodukt analog Beispiel 6 destillativ aufgearbeitet; man erhielt 38,7 g (76 %) Wertprodukt.

## Patentansprüche

1. Verfahren zur Herstellung von alpha,beta-ungesättigten beta-Oxycarbonsäurechloriden der Formel I 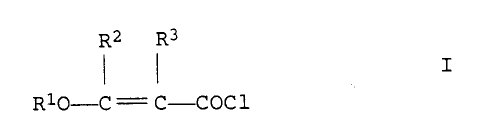 in der R¹ für eine gegebenenfalls substituierte Alkylgruppe, Cycloalkylgruppe, Alkenylgruppe, Cycloalkenylgruppe, Arylgruppe, Heterocyclylgruppe oder Heteroarylgruppe und R² und R³ unabhängig voneinander für Wasserstoff oder eine gegebenenfalls substituierte Alkylgruppe, Cycloalkylgruppe, Alkenylgruppe, Cycloalkenylgruppe, Alkinylgruppe, Arylgruppe, Heterocyclylgruppe oder Heteroarylgruppe
stehen, dadurch gekennzeichnet, daß man an ein Enolderivat der Formel II 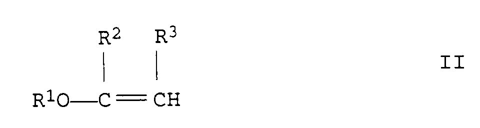 in Abwesenheit eines tertiären Amins bei 20 bis 60°C eine Verbindung IIIa, IIIb oder IIIc 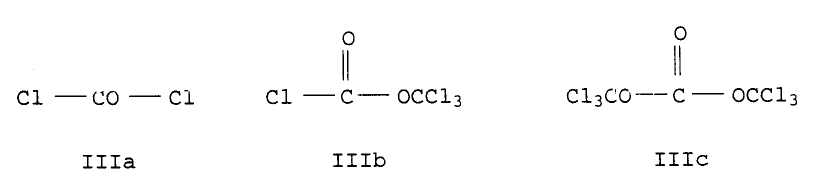 addiert und das so erhaltene Säurechlorid der Formel IV 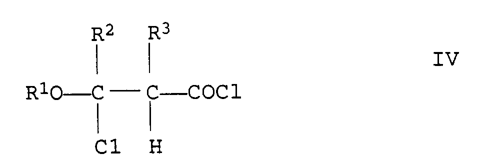 durch Eliminierung von Chlorwasserstoff (HCI) bei 30 bis 80°C zu I umsetzt.

2. Verfahren nach Anspruch 1, dadurch gekennzeichnet, daß man die Umsetzungen ohne Isolierung der Zwischenstufen (in situ) durchführt.

3. Verfahren nach Anspruch 1, dadurch gekennzeichnet, daß man den bei der Eliminierung gebildeten Chlorwasserstoff (HCl) aus dem Reaktionsgemisch entfernt.

4. Verfahren nach Anspruch 1, dadurch gekennzeichnet, daß man die Eliminierung bei vermindertem Druck durchführt.

5. Verfahren zur Herstellung von Säurechloriden der Formel IV gemäß Anspruch 1, dadurch gekennzeichnet, daß man an ein Enolderivat der Formel II 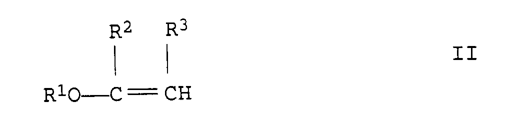 eine Verbindung IIIa, IIIb oder IIIc gemäß Anspruchs 1, addiert.

6. Verfahren nach Anspruch 1 oder 5, dadurch gekennzeichnet, daß man die Vorprodukte II und IIIa, IIIb oder IIIc in einem Verhältnis von 0,1:1 mol II/IIIa, IIIb oder IIIc bis 1:1 mol II/IIIa, IIIb oder IIIc miteinander umsetzt.

7. Verfahren nach Anspruch 1, dadurch gekennzeichnet, daß die Substituenten die folgende Bedeutung haben:
R¹ ggf. substituiertes C₁-C₈-Alkyl, C₃-C₈-Alkenyl, C₃-C₈-Alkinyl, C₃-C₈-Cycloalkyl oder C₅-C₈-Cycloalkenyl, ein ggf. substituiertes 3- bis 8-gliedriges, gesättigtes oder einfach ungesättigtes Ringsystem, welches neben Kohlenstoff-Ringgliedern ein bis drei Heteroatome aus der Gruppe Stickstoff, Sauerstoff und Schwefel enthalten kann, ggf. substituiertes Phenyl, 1-Naphthyl oder 2-Naphthyl, ein ggf. substituiertes 5-gliedriges aromatisches Ringsystem, welches neben Kohlenstoff-Ringgliedern ein Sauerstoff- oder ein Schwefelatom enthält, ein ggf. substituiertes 6-gliedriges aromatisches Ringsystem, welches neben Kohlenstoff-Ringgliedern ein bis drei Stickstoffatome enthält;
R² und R³ unabhängig voneinander Wasserstoff oder ggf. substituiertes C₁-C₈-Alkyl, C₃-C₈-Alkenyl, C₃-C₈-Alkinyl, C₃-C₈-Cycloalkyl oder C₅-C₈-Cycloalkenyl, ein ggf. substituiertes 3- bis 8-gliedriges, gesättigtes oder einfach ungesättigtes Ringsystem, welches neben Kohlenstoff-Ringgliedern ein bis drei Heteroatome aus der Gruppe Stickstoff, Sauerstoff und Schwefel enthalten kann, ggf. substituiertes Phenyl, 1-Naphthyl oder 2-Naphthyl,
ein ggf. substituiertes 5-gliedriges aromatisches Ringsystem, welches neben Kohlenstoff-Ringgliedern ein Sauerstoff- oder Schwefelatom enthält.

## Claims

1. A process for the preparation of α,β-unsaturated β-oxycarboxylic acid chlorides of the formula I 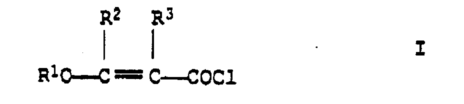 where R¹ is substituted or unsubstituted alkyl, cycloalkyl, alkenyl, cycloalkenyl, aryl, heterocyclyl or heteroaryl, and R² and R³, independently of one another, are hydrogen or substituted or unsubstituted alkyl, cycloalkyl, alkenyl, cycloalkenyl, alkynyl, aryl, heterocyclyl or heteroaryl, which comprises carrying out the addition reaction of an enol derivative of the formula II 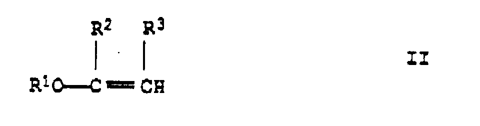 with a compound of the formula IIIa, IIIb or IIIc 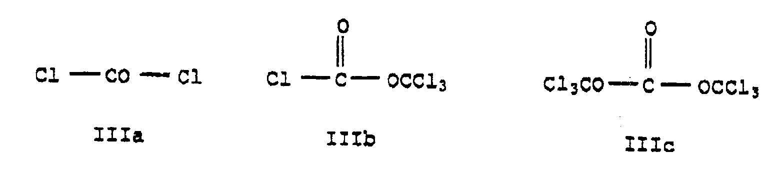 at from 20 to 60°C in the absence of a tertiary amine, and converting the resultant acid chloride of the formula IV 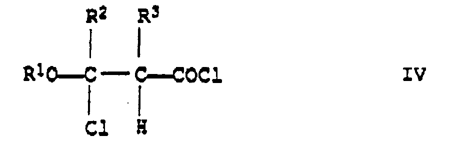 into I by elimination of hydrogen chloride (HCl) at from 30 to 80°C.

2. A process as claimed in claim 1, wherein the reactions are carried out without isolation of the intermediates (in situ).

3. A process as claimed in claim 1, wherein the hydrogen chloride (HCl) formed during the elimination is removed from the reaction mixture.

4. A process as claimed in claim 1, wherein the elimination is carried out under reduced pressure.

5. A process for the preparation of acid chlorides of the formula IV as claimed in claim 1, wherein an addition reaction is carried out between an enol derivative of the formula II 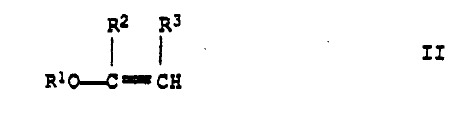 and a compound of the formula IIIa, IIIb or IIIc as defined in claim 1.

6. A process as claimed in claim 1 or 5, wherein the precursors II and IIIa, IIIb or IIIc are reacted with one another in a molar II:IIIa/IIIb/IIIc ratio of from 0.1:1 to 1:1.

7. A process as claimed in claim 1, wherein
R¹ is substituted or unsubstituted C₁-C₈-alkyl, C₃-C₈-alkenyl, C₃-C₈-alkynyl, C₃-C₈-cycloalkyl or C₅₋C₈₋ cycloalkenyl, a substituted or unsubstituted, 3- to 8-membered, saturated or monounsaturated ring system which, in addition to carbon ring members, may contain one to three heteroatoms from the group consisting of nitrogen, oxygen and sulfur, substituted or unsubstituted phenyl, 1-naphthyl or 2-naphthyl, a substituted or unsubstituted, 5-membered aromatic ring system which, in addition to carbon ring members, contains one oxygen or one sulfur atom, or a substituted or unsubstituted, 6-membered aromatic ring system which, in addition to carbon ring members, contains one to three nitrogen atoms; and
R² and R³, independently of one another, are hydrogen or substituted or unsubstituted C₁-C₈-alkyl, C₃-C₈-alkenyl, C₃-C₈-alkynyl, C₃-C₈-cycloalkyl or C₅₋C₈₋ cycloalkenyl, a substituted or unsubstituted, 3- to 8-membered, saturated or monounsaturated ring system which, in addition to carbon ring members, may contain one to three heteroatoms from the group consisting of nitrogen, oxygen and sulfur, substituted or unsubstituted phenyl, 1-naphthyl or 2-naphthyl, or a substituted or unsubstituted, 5-membered aromatic ring system which, in addition to carbon ring members, contains an oxygen or sulfur atom.

## Revendications

1. Procédé de préparation de chlorures d'acides bêta-oxycarboxyliques alpha,béta-insaturés de formule I 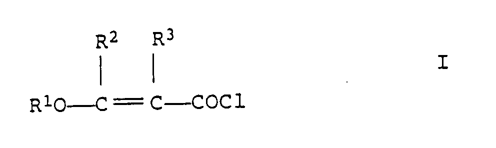 dans laquelle R¹ est mis pour un groupement alkyle éventuellement substitué, un groupement cycloalkyle, un groupement alcényle, un groupement cycloalcényle, un groupement aryle, un groupement hétérocyclique ou hétéroarylique et R² et R³ sont mis, indépendamment l'un de l'autre, pour un atome d'hydrogène ou un groupement alkyle éventuellement substitué, un groupement cycloalkyle, un groupement alcényle, un groupement cycloalcényle, un groupement alcynyle, un groupement aryle, un groupement hétérocyclique ou hétéroarylique
caractérisé en ce que l'on ajoute à un dérivé énol de formule II 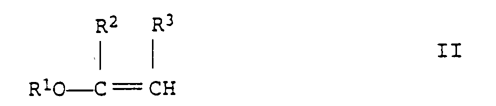 en l'absence d'amine tertiaire à 20-60°C, un composé IIIa, IIIb ou IIIc 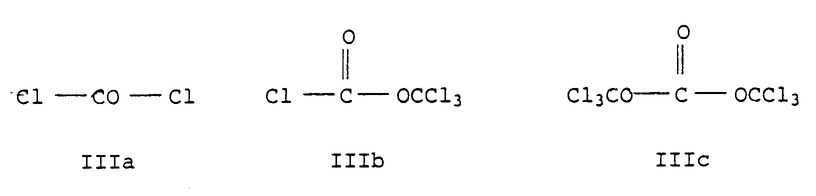 et en ce que l'on transforme le chlorure d'acide de formule IV ainsi obtenu 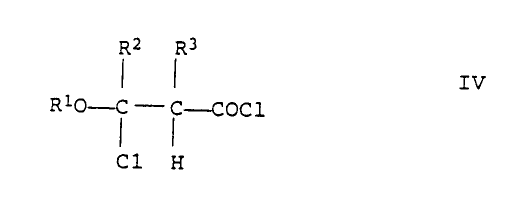 en I, par élimination de chlorure d'hydrogène (HCl) à 30-80°C.

2. Procédé selon la revendication 1, caractérisé en ce que l'on effectue les transformations sans isolement de l'étape intermédiaire (in situ).

3. Procédé selon la revendication 1, caractérisé en ce que l'on extrait du mélange réactionnel le chlorure d'hydrogène (HCl) formé lors de l'élimination.

4. Procédé selon la revendication 1, caractérisé en ce que l'on effectue l'élimination sous pression réduite.

5. Procédé de préparation de chlorures d'acides de formule IV selon la revendication 1, caractérisé en ce que l'on ajoute à un dérivé énol de formule II 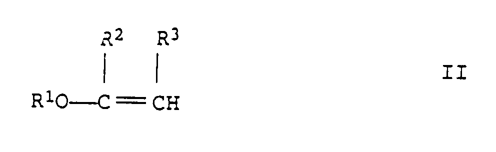 un composé IIIa, IIIb ou IIIc selon la revendication 1.

6. Procédé selon la revendication 1 ou 5, caractérisé en ce que l'on fait réagir ensemble les produits de départ II et IIIa, IIIb ou IIIc dans un rapport en moles II/IIIa, IIIb ou IIIc de 0,1:1 à 1:1.

7. Procédé selon la revendication 1, caractérisé en ce que les substituants ont la signification suivante:
R¹ groupement alkyle en C₁-C₈ éventuellement substitué, groupement alcényle en C₃-C₈, groupement alcynyle en C₃-C₈, groupement cycloalkyle en C₃-C₈ ou cycloalcényle en C₅-C₈, un système cyclique, éventuellement substitué, à 3-8 maillons saturé ou ayant une insaturation, qui peut contenir un à trois hétèroatomes choisis dans le groupe formé par l'azote, l'oxygène et le soufre en plus des atomes de carbone du cycle, un groupement phényle éventuellement substitué, un groupement 1-naphtyle ou 2-naphtyle, un système cyclique aromatique à 5 maillons, éventuellement substitué, qui contient un atome d'oxygène ou de soufre en plus des atomes de carbone du cycle, un système cyclique aromatique à 6 maillons, éventuellement substitué, qui contient 1 à 3 atomes d'azote en plus des atomes de carbone du cycle ;
R² et R³ indépendamment l'un de l'autre, atome d'hydrogène ou groupement alkyle en C₁-C₈ éventuellement substitué, groupement alcényle en C₃-C₈, groupement alcynyle en C₃-C₈, groupement cycloalkyle en C₃-C₈ ou cycloalcényle en C₅-C₈, un système cyclique, éventuellement substitué, à 3-8 maillons, saturé ou ayant une insaturation, qui peut contenir un à trois hétéroatomes choisis dans le groupe formé par l'azote, l'oxygène et le soufre en plus des atomes de carbone du cycle, un groupement phényle éventuellement substitué, un groupement 1-naphtyle ou 2-naphtyle, un système cyclique aromatique à 5 maillons, éventuellement substitué, qui contient un atome d'oxygène ou de soufre en plus des atomes de carbone du cycle.
